Europäisches Patentamt

(19) European Patent Office

Office européen des brevets

(11) Numéro de publication : **0 295 986 B1**

(12) **FASCICULE DE BREVET EUROPEEN**

(45) Date de publication du fascicule du brevet :
17.07.91 Bulletin 91/29

(51) Int. Cl.⁵ : **G01T 1/164, G01T 1/17**

(21) Numéro de dépôt : **88401267.5**

(22) Date de dépôt : **25.05.88**

(54) **Dispositif de localisation d'un rayonnement nucléaire, et dispositif de formation d'image de rayonnement incluant un tel dispositif de localisation.**

(30) Priorité : 27.05.87 FR 8707482

(43) Date de publication de la demande :
21.12.88 Bulletin 88/51

(45) Mention de la délivrance du brevet :
17.07.91 Bulletin 91/29

(84) Etats contractants désignés :
DE GB NL

(56) Documents cités :
FR-A- 2 546 633
US-A- 3 950 648
US-A- 4 179 607
US-A- 4 223 388

(73) Titulaire : COMMISSARIAT A L'ENERGIE ATOMIQUE Etablissement de Caractère Scientifique Technique et Industriel 31/33, rue de la Fédération F-75015 Paris (FR)

(72) Inventeur : Tournier, Edmond
2 Place Docteur Léon Martin
F-38000 Grenoble (FR)
Inventeur : Tararine, Michel
6, rue du Four
F-92330 Sceaux (FR)

(74) Mandataire : Mongrédien, André et al c/o BREVATOME 25, rue de Ponthieu F-75008 Paris (FR)

EP 0 295 986 B1

EP 0 295 986 B1

## Description

La présente invention a pour objet un dispositif de localisation d'un rayonnement nucléaire. Elle a également pour objet un dispositif de formation d'une image de rayonnement comportant un tel dispositif de localisation.

Elle trouve son application notamment dans le domaine médical où des dispositifs de formation d'image par rayonnement sont utilisés comme aide au diagnostic. De tels dispositifs sont appelés généralement caméra à scintillation, caméra à rayons gamma, ou caméra de type ANGER.

Cette application implique l'administration par injection dans une veine d'un patient d'une petite dose d'un radio-isotope (une substance radioactive qui émet des rayons gamma). La circulation sanguine distribue la dose dans le corps et un transducteur d'une sensibilité appropriée enregistre le développement de cette distribution.

Les régions du corps qui ont une grande affinité pour l'isotope ou une irrigation sanguine riche apparaissent comme des sources brillantes ou fortement éclairées tandis qu'inversement, les régions de faible affinité ou de faible irrigation sanguine apparaissent sombres. De cette manière, toute partie du corps ou un organe spécifique peut être soumis à une investigation clinique d'une manière sûre, fiable et sans intervention.

Une caméra à rayons gamma comprend une tête de détection comprenant généralement un collimateur pour focaliser les rayons gamma émis par le patient, un cristal pour transformer les photons gamma en photons lumineux, ou scintillations, et un réseau de tubes photomultiplicateurs pour transformer chaque scintillation en une impulsion électrique analogique, appelée aussi contribution électrique.

Elle comprend en outre un codeur de position pour produire des signaux de coordonnées X et Y du lieu où s'est produite une scintillation, à partir des impulsions électriques analogiques fournies par les tubes photomultiplicateurs.

La tête de détecteur et le codeur de position constituent ensemble un dispositif de localisation d'un rayonnement gamma.

Une caméra à rayons gamma comprend en outre généralement un oscilloscope cathodique commmandé par les signaux de coordonnées X et Y, et par un signal de validation Z produit par le codeur de position à partir des impulsions fournies par les tubes photomultiplicateurs, lorsque l'énergie de la scintillation appartient à une bande d'énergie prédéterminée. Un rayonnement gamma atteignant le cristal est ainsi visualisé par un point lumineux sur l'écran de l'oscilloscope cathodique.

Pour produire une image de rayonnement représentatrice de tous les rayonnement nucléaires reçus, une caméra à rayons gamma peut également comporter un dispositif photographique pour former une image de l'organe observé par accumulation d'un grand nombre de points lumineux produits sur l'écran de l'oscilloscope cathodique.

Le moyen de visualisation peut enfin comprendre un moyen de traitement numérique des images, notamment en vue d'obtenir des tomographies de l'organe observé. Pour atteindre ce but, on acquiert plusieurs images de l'organe selon une pluralité d'orientations d'observations de la caméra à rayons gamma par rapport à cet organe par des traitements du signal analogues à ceux rencontrés dans les tomodensitomètres, on peut reconstituer des images des coupes des organes examinés.

La tête de détection d'un dispositif de localisation d'un rayonnement nucléaire ou d'une caméra à rayons gammas présente, de par sa structure même, au moins deux types de défauts :

– un défaut de spectrométrie dû à la dérive du gain des tubes photomultiplicateurs dans le temps et à la non-uniformité de la sensibilité de détection desdits tubes photomultiplicateurs,

– un défaut de linéarité dû aux distorsions géométriques introduites par les photomultiplicateurs.

Ces défauts sont corrigés, de manière connue (cf. documents US-A-3745345, FR-A-2412856 et EP-A-0021366), en deux phases. Une première phase au cours de laquelle l'état de la tête de détection avec ses défauts est enregistré et comparé à une tête de détection idéale ; cette comparaison permet de calculer des coefficients de correction numériques qui sont stockés dans une mémoire. Une seconde phase au cours de laquelle les coefficients de correction mémorisés sont appliqués aux signaux de coordonnées X, Y délivrés par le codeur de position. Les signaux de coordonnées X, Y sont donc numérisés pour pouvoir être combinés aux coefficients de correction numériques.

Les signaux de coordonnées X, Y délivrés par le codeur de position lorsqu'un rayonnement gamma atteint le cristal sont produits de la manière suivante.

On sait qu'une scintillation est vue simultanément par plusieurs tubes photomultiplicateurs, de l'ordre de 6 à 10 tubes. La détermination de l'emplacement de la scintillation est obtenue en calculant l'emplacement du barycentre des contributions électriques délivrées par l'ensemble des tubes photomultiplicateurs excités par cette scintillation.

Ce calcul est effectué classiquement, comme décrit dans le document US-A-3011057, en utilisant plusieurs

2

jeux de résistances électriques dont les valeurs sont fonction des positions des tubes photomultiplicateurs auxquelles elles sont raccordées. Ces positions sont définies par rapport à un repère de référence d'axes Ox et Oy. Dans chaque jeu de résistances, il y a une résistance par tube photomultiplicateur, chaque résistance étant reliée par une extrémité à un tube photomultiplicateur et par une autre extrémité à un point commun. Le signal délivré sur ce point commun est ainsi une pondération des signaux délivrés par les tubes photomultiplicateurs.

Un codeur de position comprend généralement 4 jeux différents de résistances qui délivrent des signaux analogiques notés $X^+$, $X^-$, $Y^+$ et $Y^-$. Il peut éventuellement comprendre un cinquième jeu de résistances pour délivrer le signal de validation Z, représentatif de l'énergie de la scintillation.

Les signaux de coordonnées X, Y sont déduits, par un calcul analogique, des signaux $X^+$, $X^-$, $Y^+$, $Y^-$ et éventuellement du signal Z.

Les relations généralement utilisées pour le calcul de la coordonnée X sont l'une des suivantes :

$$X = \frac{X^+ - X^-}{X^+ + X^-} \quad \text{ou} \quad X = \frac{X^+ - X^-}{X^+ + X^- + Y^+ + Y^-} \quad \text{ou} \quad X = \frac{X^+ - X^-}{Z}$$

Des relations symétriques sont utilisées pour le calcul de la coordonnée Y.

Ces calculs employant des diviseurs analogiques, il est nécessaire de normaliser les signaux $X^+$, $X^-$, $Y^+$, $Y^-$ en fonction du signal d'énergie Z afin de placer le diviseur dans sa gamme de linéarité.

Les signaux de coordonnées X, Y obtenus, et le signal Z, le cas échéant, sont ensuite appliqués sur les entrées de convertisseurs analogiques-numériques pour être numérisés.

Cette technique présente l'inconvénient de nécessiter des convertisseurs analogiques-numériques ayant une excellente performance de linéarité différentielle.

En effet, si la gamme d'amplitude pour les signaux de coordonnées X, Y est découpée par chaque convertisseur analogique-numérique en segments de largeurs non-identiques, certaines valeurs numériques des coordonnées X, Y seront favorisées aux dépens d'autres valeurs numériques.

Considérons, à titre d'exemple, le cas d'un signal analogique dont l'amplitude est comprise dans la gamme 0,99 et dont la valeur est codée sur 2 bits. Un convertisseur analogique numérique parfait fait correspondre la valeur logique 00 au segment [0, 24], la valeur logique 01 au segment [25, 49], la valeur logique 10 au segment [50, 74] et la valeur logique 11 au segment [75, 99]. Tous les segments ont la même longueur.

En revanche, un convertisseur analogique-numérique imparfait associe, par exemple, la valeur logique 00 au segment [0, 27], la valeur logique 01 au segment [28, 49], la valeur logique 10 au segment [50, 74] et la valeur logique 11 au segment [75, 99]. Ainsi, un signal analogique de coordonnées d'amplitude 26 sera codé par la valeur logique 00 avec le convertisseur imparfait, au lieu d'être codé par la valeur logique 01 avec le convertisseur parfait. Il s'ensuit une erreur dans la localisation de la scintillation.

De même, dans le cas d'un dispositif de formation d'une image de rayonnement, une image de rayonnement normalement uniforme apparaîtra sur un écran avec des lignes ou des colonnes de densité supérieure ou inférieure à la moyenne selon que le segment associé à ladite ligne ou à ladite colonne est moins large ou plus large que la moyenne.

Un tel phénomène est extrêmement visible, l'oeil étant très sensible aux variations de contraste même très légères, si ces variations ont une forme géométrique.

On pourra également se reporter à l'art antérieur constitué par le brevet US-A-4179607. Cependant, l'invention décrite dans ce brevet concerne la non-uniformité ou le manque de correspondance entre les positions de scintillation et les coordonnées X, Y calculées et affichées. Toutefois, ce brevet n'apporte aucun enseignement sur la façon d'obtenir les positions de scintillation et ne permet donc pas de résoudre le problème de la linéarité différentielle des codeurs de position, tel que le propose la présente invention.

On pourra aussi se reporter au brevet US-A-4223388. Mais, comme dans le brevet précédent, le problème posé n'est pas le même que celui qui est posé et résolu dans la présente demande.

L'invention a notamment pour but d'obtenir les signaux de coordonnées X, Y sous forme numérique au moyen de convertisseurs analogiques-numériques n'ayant pas nécessairement une très grande linéarité différentielle.

L'invention consiste à numériser les signaux $X^+$, $X^-$, $Y^+$, $Y^-$ (et, le cas échéant Z) et à produire des signaux de coordonnées numériques X, Y à partir desdits signaux numérisés. De cette manière, la linéarité différentielle des convertisseurs analogiques-numériques qui délivrent les signaux numériques $X^+$, $X^-$, $Y^+$, $Y^-$ et Z n'est pas un paramètre critique car dans l'image finale, un même signal de coordonnée numérique X est obtenu par un grand nombre de couples de signaux numériques $(X_N^+, X_N^-)$ différents correspondant à la fluctuation statistique de l'énergie des scintillations.

De manière précise, l'invention a pour objet un dispositif de localisation d'un rayonnement nucléaire comprenant une tête de détection pour détecter un rayonnement nucléaire et un codeur de position pour localiser ledit rayonnement nucléaire, ladite tête de détection comprenant :

– un collimateur,

– une couche plane d'un matériau sensible audit rayonnement nucléaire pour recevoir sur une première face, ou face avant, un rayonnement nucléaire traversant ledit collimateur et pour délivrer sur une seconde face, ou face arrière, un rayonnement lumineux fonction du rayonnement nucléaire reçu,

– un ensemble de transducteurs disposés sur la face arrière de ladite couche, délivrant chacun un signal électrique fonction du rayonnement lumineux reçu, chaque rayonnement nucléaire produisant un rayonnement lumineux vu par une pluralité de transducteurs,

ledit dispositif de localisation étant caractérisé en ce que ledit codeur de position comprend :

— des moyens de pondération pour délivrer des signaux électriques analogiques $X^+$, $X^-$, $Y^+$ et $Y^-$ représentant chacun des sommes pondérées des signaux électriques délivrés par les transducteurs, les signaux $X^+$, $X^-$ étant fonction de la position et de l'énergie du rayonnement nucléaire reçu suivant un axe X, et les signaux $Y^+$, $Y^-$ étant fonction de la position du rayonnement nucléaire reçu suivant un axe Y,

— un moyen de numérisation pour recevoir les signaux analogiques $X^+$, $X^-$, $Y^+$ et $Y^-$ et délivrer des signaux numériques correspondants $X_N^+$, $X_N^-$, $Y_N^+$ et $Y_N^-$,

— un moyen de calcul pour déterminer des signaux de coordonnées numériques $X_N$, $Y_N$ du rayonnement nucléaire reçu en fonction des signaux numériques $X_N^+$, $X_N^-$, $Y_N^+$ et $Y_N^-$.

L'invention a également pour objet un dispositif de formation d'image de rayonnement comprenant un tel dispositif de localisation, et un moyen d'affichage comprenant un moyen de visualisation du point d'image de coordonnées $X_N$, $Y_N$ et/ou un moyen imageur pour accumuler lesdits points d'image.

Les caractéristiques et avantages de l'invention ressortiront mieux de la description qui va suivre, donnée à titre illustratif mais non limitatif, en référence aux dessins annexés, sur lesquels :

– la figure 1 illustre le défaut d'affichage produit par les dispositifs de formation d'image de rayonnement selon l'art antérieur, lorsque les convertisseurs analogiques-numériques du codeur de position présentent une non-linéarité différentielle, même faible,

– la figure 2 représente schématiquement la structure générale d'un dispositif de localisation de rayonnement nucléaire,

– la figure 3 montre, en vue de dessus, l'arrangement des tubes photomultiplicateurs sur le cristal de la tête de détection d'un dispositif de localisation de rayonnement nucléaire,

– la figure 4 est un diagramme illustrant l'amplitude des signaux $X^+$, $X^-$ en fonction de la position du tube photomultiplicateur selon l'axe Ox,

– la figure 5 est un histogramme de l'énergie d'une scintillation,

– la figure 6 illustre le découpage d'une gamme d'amplitude en segments inégaux par un convertisseur analogique-numérique présentant une non-linéairté différentielle,

– la figure 7 représente un codeur de position selon un premier mode de réalisation de l'invention,

– la figure 8 représente un codeur de position selon un second mode de réalisation de l'invention, et

– la figure 9 représente schématiquement un dispositif de formation d'image de rayonnement selon l'invention.

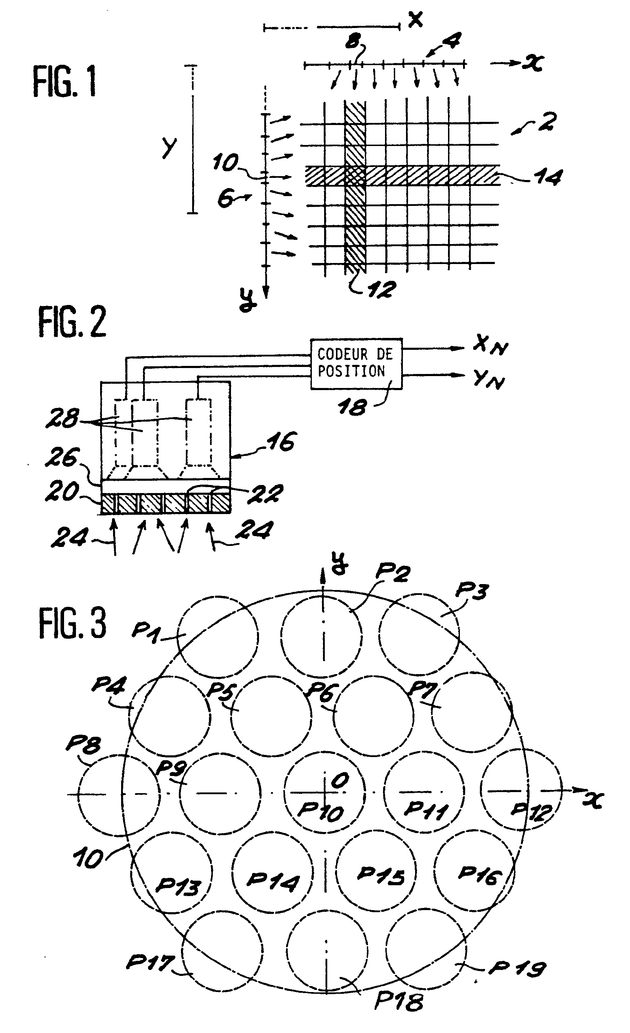

On a représenté sur la figure 1 une image numérique 2 composée d'un ensemble de points d'image, ou pixels, arrangés en matrice. Cette image correspond à une image de rayonnement uniforme reçue par un dispositif de formation d'image de rayonnement selon l'art antérieur, dont le codeur de position produit des signaux de coordonnées X, Y analogiques qui sont ensuite numérisés au moyen de convertisseurs analogiques-numériques présentant une non-linéarité différentielle.

Les caractéristiques des convertisseurs analogiques-numériques pour les signaux de coordonnées X, Y ont été représentés respectivement par les références numériques 4, 6. Ces caractéristiques associent une valeur numérique (numéro de colonne ou numéro de ligne d'image numérique) à un signal de coordonnée X, Y en fonction de l'amplitude dudit signal.

Si les convertisseurs analogiques-numériques étaient parfaits, une gamme d'amplitude, ou segment, de même longueur serait affectée à chaque ligne (ou colonne) de l'image numérique. Au contraire, un segment 8, 10 plus court contiendra moins de scintillations que les autres segments (pour une image de rayonnement uniforme) ce qui se traduit, pour la colonne 12 ou la ligne 14 associée, par une intensité différente de celles des autres lignes et colonnes.

L'invention vise à supprimer ou au moins fortement atténuer, un tel défaut.

L'invention a pour objet un dispositif de localisation de rayonnement nucléaire tel que représenté schématiquement sur la figure 2.

Ce dispositif de localisation comprend une tête de détection 16 et un codeur de position 18. La tête de détection comprend généralement de manière connue un collimateur 20 percé d'une multitude de trous parallèles 22 pour que seul un rayonnement nucléaire 24, constitué par exemple de photons gamma, provenant d'une direction déterminée soit reçu. Ce rayonnement nucléaire est converti en photons lumineux ou scintillations, dans une couche 26 d'un matériau de type cristal de NaI(Tl). Les photons lumineux sont reçus dans un ensemble de tubes photomultiplicateurs 28 qui délivrent chacun un signal électrique appelé contribution électrique dont l'amplitude est fonction de la localisation des scintillations dans le cristal.

Les tubes photomultiplicateurs sont disposés de manière à recouvrir au mieux la surface du cristal 26 comme indiqué sur la vue de dessus de la figure 3. Chaque tube photomultiplicateur est entouré de six tubes photomultiplicateurs. Le nombre total de tubes photomultiplicateurs est en général de 19, 37, 61 ou 93. Ce nombre dépend de la taille des tubes photomultiplicateurs et de la surface du cristal.

Les contributions électriques de chaque tube photomultiplicateur sont reçues par le codeur de position 18 (figure 2). De manière connue (cf US-A-3011057) des jeux de résistances électriques sont utilisés pour produire des signaux analogiques $X^+$, $X^-$, $Y^+$, $Y^-$. Chaque jeu de résistances comprend une résistance par tube photomultiplicateur dans lequel les valeurs relatives des résistances sont fonction de la position du tube photomultiplicateur associé, dans un repère xOy généralement centré sur le centre du cristal (cf figure 3).

Les signaux analogiques $X^+$, $X^-$, $Y^+$, $Y^-$ forment ainsi des sommes pondérées des contributions électriques des tubes photomultiplicateurs.

Un jeu de résistances supplémentaires peut être utilisé, dans lequel toutes les résistances ont une valeur identique, pour sommer les contributions électriques des tubes photomultiplicateurs et produire ainsi un signal Z représentatif de l'énergie de la scintillation.

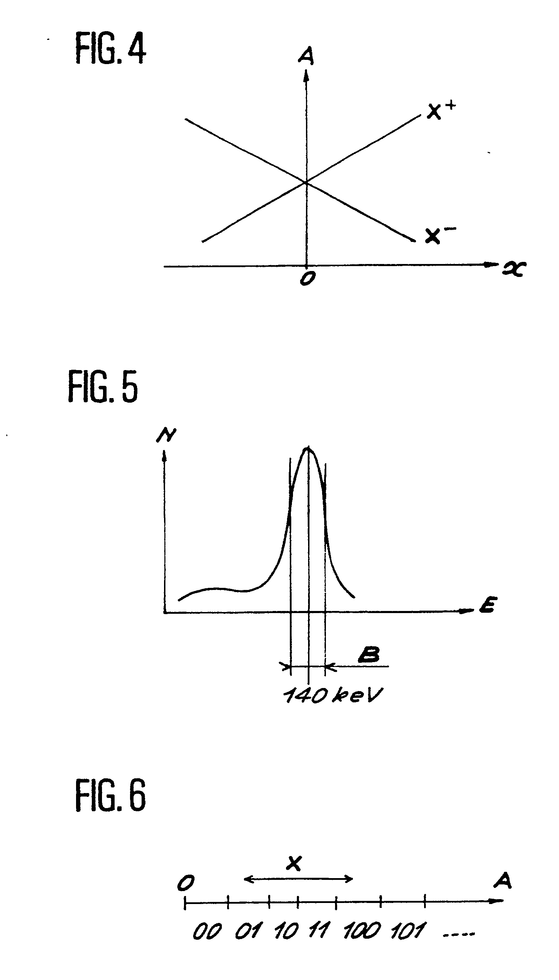

On a représenté sur la figure 4 un diagramme de l'amplitude A des signaux $X^+$ et $X^-$ en fonction de la position de la scintillation selon l'axe Ox. Les signaux $Y^+$ et $Y^-$ ont une amplitude analogue en fonction de la position de la scintillation selon l'axe Oy.

Selon l'invention, les signaux $X^+$, $X^-$, $Y^+$, $Y^-$ et éventuellement le signal Z sont d'abord numérisés, puis ensuite combinés pour produire les signaux de coordonnées X, Y numériques. Ceci permet d'éviter plus facilement, ou au moins d'atténuer fortement, les défauts de visualisation rencontrés dans l'art antérieur et mentionnés en référence à la figure 1.

L'avantage apporté par l'invention trouve son origine dans la statistique de l'énergie des photons gamma reçus par le cristal. Ceci va être expliqué en relation avec les figures 5 et 6.

La figure 5 est un histogramme de l'énergie des photons gamma reçus par le cristal. L'énergie théorique d'un photon gamma est de 140 keV dans le cas ou on utilise comme élément radioactif l'élément $^{99}$Tc. En pratique l'énergie du photon gamma est située dans une bande étroite B centrée sur l'énergie théorique, et de largeur à mi-hauteur environ 10% de la valeur de l'énergie théorique. Quelques photons gamma de faible énergie sont également émis par diffusion Compton.

Cette distribution énergétique des photons gamma dans la bande B implique que deux photons, arrivant au même endroit sur le cristal mais ayant une énergie différente, exciteront différemment les tubes photomultiplicateurs et produiront donc des signaux $X^+$, $X^-$, $Y^+$ et $Y^-$ différents.

Un même point d'image de coordonnées X, Y pourra donc être obtenu à partir de signaux $X^+$, $X^-$, $Y^+$ et $Y^-$ différents.

La fluctuation statistique réalise un lissage qui masque la non-linéarité différentielle éventuelle des convertisseurs analogiques-numériques.

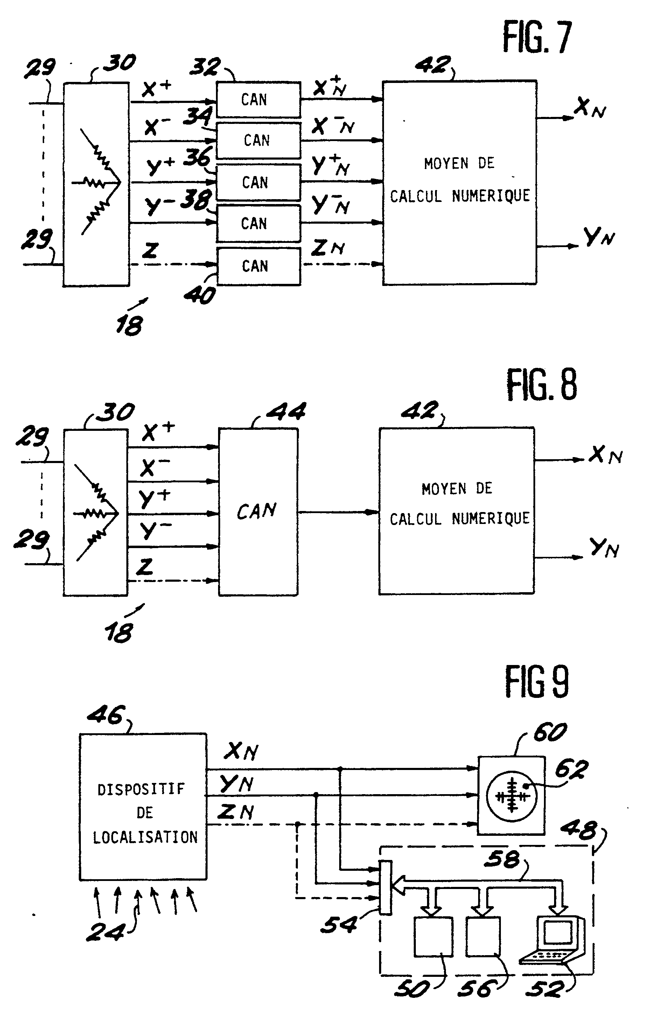

La conversion analogique-numérique est illustrée schématiquement sur la figure 7. Des valeurs numériques, exprimées sous forme binaire, 00, 01, 10, 11, 100, 101,... sont associées à des gammes d'amplitudes représentées par des segments sur un axe OA notant l'amplitude d'un signal analogique.

La valeur 10 est associée à un segment plus court que les autres valeurs pour indiquer une non-linéarité différentielle du convertisseur analogique-numérique.

La fluctuation statistique de l'énergie des photons gamma a pour conséquence qu'un même couple de coordonnées X, Y est obtenu pour un ensemble d'amplitudes des signaux $X^+$, $X^-$, $Y^+$, $Y^-$, chaque ensemble d'amplitudes formant une gamme recouvrant plusieurs gammes du convertisseur analogique-numérique, comme on l'a représenté sur la figure 6 pour le signal $X^+$.

Le défaut local représenté par le segment court associé à la valeur 10 est ainsi noyé dans un segment plus long créé par la fluctuation statistique.

On a constaté expérimentalement que le fait de numériser d'abord les signaux $X^+$, $X^-$, $Y^+$, $Y^-$, conformément à l'invention, permettait d'obtenir avec des convertisseurs analogiques-numériques à 8 bits une image numérique de qualité comparable à celle obtenue, selon la technique antérieure où les signaux de coordonnées X, Y sont calculés analogiquement, puis numérisés, avec des convertissseurs analogiques-numériques à 12 bits. De plus, dans l'invention, la contrainte de linéarité différentielle est moins forte. A titre d'exemple, des mesures expérimentales ont montré que l'amélioration de la linéarité dans l'image finale obtenue pourrait atteindre un facteur 20.

L'invention permet donc de réduire notablement le coût de la partie codeur de position d'un dispositif de localisation ou d'un dispositif de formation d'une image de rayonnement.

On a représenté respectivement sur les figures 7 et 8 des modes de réalisation d'un codeur de position conforme à l'invention.

Le codeur de position 18 représenté sur la figure 7 comprend un moyen de pondération 30, de type connu, constitué d'un ensemble de résistances pour délivrer les signaux analogiques $X^+$, $X^-$, $Y^+$, $Y^-$ et éventuellement Z, comme sommes pondérées des contributions électriques reçues des photomultiplicateurs par des lignes 29. Les signaux analogiques sont ensuite appliqués sur les entrées de convertisseurs analogiques-numériques 32-40 qui délivrent des signaux numériques $X_N^+$, $X_N^-$, $Y_N^+$, $Y_N^-$ et $Z_N$ correspondants.

Ces signaux sont reçus dans un moyen de calcul numérique 42 dans lequel ils sont combinés pour produire les signaux numériques de coordonnées $X_N$, $Y_N$. Ce moyen de calcul peut être réalisé de toute manière connue, soit par un ensemble de circuits logiques, soit sous la forme d'un programme d'ordinateur.

Le signal numérique de coordonnée $X_N$ est produit de préférence suivant l'une des relations suivantes :

$$X_N = \frac{X_N^+ - X_N^-}{X_N^+ + X_N^-} \quad ou \quad X_N = \frac{X_N^+ - X_N^-}{X_N^+ + X_N^- + Y_N^+ + Y_N^-} \quad ou \quad X_N = \frac{X_N^+ - X_N^-}{Z_N}$$

et, symétriquement, le signal de coordonnée $Y_N$ est produit de préférence suivant l'une des relations suivantes :

$$Y_N = \frac{Y_N^+ - Y_N^-}{Y_N^+ + Y_N^-} \quad ou \quad Y_N = \frac{Y_N^+ - Y_N^-}{Y_N^+ + Y_N^- + X_N^+ + X_N^-} \quad ou \quad Y_N = \frac{Y_N^+ - Y_N^-}{Z_N}$$

La figure 8 illustre une variante de réalisation du codeur numérique dans lequel l'ensemble des convertisseurs analogiques-numériques 32-40 est remplacé par un unique convertisseur analogique-numérique 44 multiplexé. Ce convertisseur 44 peut être commandé de toute manière connue, notamment par un signal appliqué sur une entrée de commande et spécifiant l'entrée sélectionnée, ou par un signal d'horloge qui sélectionne les entrées de manière cyclique.

Le moyen de pondération 30 et le moyen de calcul numérique 42 sont fonctionnellement identiques sur les figures 7 et 8.

On a décrit en référence aux figures 2 à 8 un dispositif de localisation de rayonnement nucléaire selon l'invention.

Ce dispositif de localisation est souvent associé à un moyen de formation d'image pour former un dispositif de formation d'image de rayonnement, comme on l'a représenté sur la figure 9.

Sur cette figure, les signaux numériques de coordonnées $X_N$, $Y_N$ délivrés par le dispositif de localisation 46, en réponse à un rayonnement nucléaire reçu, sont transmis à un moyen de formation d'image, ou imageur 48.

Cet imageur comprend une mémoire 50 pour mémoriser le nombre de rayonnements reçus en chaque point d'image, un afficheur 52, pour visualiser l'image contenue dans la mémoire, un circuit d'entrée-sortie 54 pour recevoir les signaux numériques de coordonnées $X_N$, $Y_N$, et un moyen de traitement 56 pour commander, par une voie 58, la mémoire 50, l'afficheur 52 et le circuit d'entrée-sortie 54.

Le moyen de traitement 56 est conçu pour commander la visualisation sur l'afficheur 52 d'une image contenue dans la mémoire 50. Il peut être conçu en outre pour faire des traitements mathématiques sur les images contenues dans la mémoire 50, notamment en vue d'obtenir des tomographies.

Le dispositif de formation d'image de rayonnement, ou le dispositif de localisation de rayonnement, peut comporter en outre un moyen de visualisation 60, par exemple de type oscilloscope cathodique pour visualiser la position 62 du point d'image correspondant au couple de coordonnées $(X_N, Y_N)$ reçu.

La visualisation d'un point d'image sur le moyen de visualisation ou la prise en compte dans la mémoire d'un point d'image est généralement soumise à une condition sur l'amplitude du signal Z représentatif de l'énergie de la scintillation.

**Revendications**

1. Dispositif de localisation d'un rayonnement nucléaire comprenant un tête de détection (16) pour détecter un rayonnement nucléaire (24) et un codeur de position (18) pour localiser ledit rayonnement nucléaire, ladite tête de détection comprenant :
— un collimateur (20),
— une couche plane (26) d'un matériau sensible audit rayonnement nucléaire pour recevoir sur une première face, ou face avant, un rayonnement nucléaire traversant ledit collimateur et pour délivrer sur une seconde face, ou face arrière, un rayonnement lumineux fonction du rayonnement nucléaire reçu,
— un ensemble de transducteurs (28) disposés sur la face arrière de ladite couche, délivrant chacun un signal électrique fonction du rayonnement lumineux reçu, chaque rayonnement nucléaire produisant un rayonnement lumineux vu par une pluralité de transducteurs, dispositif dans lequel ledit codeur de position (18) comprend :
— des moyens de pondération (30) pour délivrer des signaux électriques analogiques $X^+$, $X^-$, $Y^+$ et $Y^-$ représentant chacun des sommes pondérées des signaux électriques délivrés par les transducteurs, les signaux $X^+$, $X^-$ étant fonction de la position et de l'énergie du rayonnement nucléaire reçu suivant un axe X, et les signaux $Y^+$, $Y^-$ étant fonction de la position du rayonnement nucléaire reçu suivant un axe Y ; caractérisé en ce qu'il comprend en outre :
— un moyen de numérisation (32-38, 44) pour recevoir les signaux analogiques $X^+$, $X^-$, $Y^+$ et $Y^-$ et délivrer des signaux numériques correspondants $X_N^+$, $X_N^-$, $Y_N^+$ et $Y_N^-$,
— un moyen de calcul numérique (42) pour déterminer des signaux de coordonnées numériques $X_N$, $Y_N$ du rayonnement nucléaire reçu en fonction des signaux numérisés $X_N^+$, $X_N^-$, $Y_N^+$ et $Y_N^-$.

2. Dispositif selon la revendication 1, caractérisé en ce que le moyen de numérisation comprend 4 convertisseurs analogiques-numériques (32-38) traitant chacun l'un des signaux analogiques $X^+$, $X^-$, $Y^+$, $Y^-$.

3. Dispositif selon la revendication 1, caractérisé en ce que le moyen de numérisation comprend un convertisseurs analogique-numérique multiplexé (44) pour traiter séquentiellement les signaux analogiques $X^+$, $X^-$, $Y^+$, $Y^-$.

4. Dispositif selon l'une quelconque des revendications 1 à 3, caractérisé en ce que le moyen de calcul numérique (42) produit les signaux numériques de coordonnées $X_N$, $Y_N$ selon les relations :

$$X_N = \frac{X_N^+ - X_N^-}{X_N^+ + X_N^-} \quad \text{et} \quad Y_N = \frac{Y_N^+ - Y_N^-}{Y_N^+ + Y_N^-}$$

5. Dispositif selon l'une quelconque des revendications 1 à 3, caractérisé en ce que le moyen de calcul numérique (42) produit les signaux numériques de coordonnées $X_N$, $Y_N$ selon les relations :

$$X_N = \frac{X_N^+ - X_N^-}{X_N^+ + X_N^- + Y_N^+ + Y_N^-} \quad \text{et} \quad Y_N = \frac{Y_N^+ - Y_N^-}{X_N^+ + X_N^- + Y_N^+ + Y_N^-}$$

6. Dispositif selon l'une quelconque des revendications 1 à 3, caractérisé en ce que le codeur de position (8) comprend en outre un moyen de pondération (30) pour délivrer un signal analogique d'énergie Z égal à la somme des signaux électriques délivrés par les transducteurs (28), ledit signal Z étant converti en un signal numérique $Z_N$ par le moyen de numérisation du codeur de position.

7. Dispositif selon la revendication 6, caractérisé en ce que le moyen de calcul numérique (42) produit les signaux numériques de coordonnées $X_N$, $Y_N$ selon les relations :

$$X_N = \frac{X_N^+ + X_N^-}{Z_N} \quad et \quad Y_N = \frac{Y_N^+ - Y_N^-}{Z_N}$$

8. Dispositif de formation d'image de rayonnement comprenant un dispositif de localisation d'un rayonnement nucléaire (46) délivrant des signaux numériques de coordonnées $X_N$, $Y_N$ indiquant la position d'un rayonnement nucléaire reçu, et un moyen de formation d'image (48) comprenant une mémoire, un afficheur et un moyen de traitement recevant lesdits signaux numériques de coordonnées $X_N$, $Y_N$, stockant dans ladite mémoire le nombre de rayonnements nucléaires reçus en chaque point d'image de coordonnées $X_N$, $Y_N$ et commandant l'afficheur pour produire une image de rayonnement dans laquelle l'intensité de chaque point d'image est fonction dudit nombre de rayonnements nucléaires reçus, caractérisé en ce que ledit dispositif de localisation d'un rayonnement nucléaire est conforme à l'une quelconque des revendications 1 à 7.

## Patentansprüche

1. Vorrichtung zur Kern-Strahlungslokalisierung mit einem Detektorkopf (16) zum Auffinden einer Kernstrahlung (24) und mit einem Positionskodierer (18) zum Lokalisieren dieser Kernstrahlung, wobei der Detektorkopf aufweist :
— eine Richteinrichtung (20),
— eine ebene Schicht (26) aus einem Material, das für eine solche Kernstrahlung empfindlich ist, um auf einer ersten Fläche oder einer vorderen Fläche eine Kernstrahlung, die die Richteinrichtung (20) passiert hat, und um auf einer zweiten Fläche oder einer hinteren Fläche eine Lichtstrahlung zu empfangen, die von der empfangenen Kernstrahlung abhängt,
— eine Anordnung von Meßwertumsetzern (28), die auf der hinteren Fläche der Schicht angeordnet sind und jeweils ein elektrisches Signal in Abhängigkeit von der empfangenen Lichtstrahlung abgeben, wobei jede Kernstrahlung eine über eine Vielzahl von Meßwertumsetzern zu sehende Lichtstrahlung erzeugt,
— wobei in dieser Vorrichtung der Positionskodierer (18) aufweist :
— Bewertungseinrichtungen (30) zum Abgeben von elektrischen Analog-Signalen $X^+$, $X^-$, $Y^+$, und $Y^-$, die jeweils Summen darstellen, die aus der Bewertung der von den Meßwertumsetzern gelieferten elektrischen Signale hervorgegangen sind, wobei die Signale $X^+$, $X^-$ von der Position und der Energie der entlang einer Achse X empfangenen Kernstrahlung und die Signale $Y^+$, $Y^-$ von der Stellung der entlang einer Achse Y empfangenen Kernstrahlung abhängen ; **gekennzeichnet dadurch, daß** sie außerdem aufweist :
— eine Digitalisierseinrichtung (32-38, 44) zum Empfangen von Analogsignalen $X^+$, $X^-$, $Y^+$, und $Y^-$ und zum Abgeben von entsprechenden digitalen Signalen $X_N^+$, $X_N^-$, $Y_N^+$, und $Y_N^-$,
— eine Digital-Recheneinrichtung (42) zum Bestimmen von digitalen Koordinaten-Signalen $X_N$, $Y_N$ der empfangenen Kernstrahlung in Abhängigkeit von den digitalen Signalen $X_N^+$, $X_N^-$, $Y_N^+$, und $Y_N^-$.

2. Vorrichtung nach Anspruch 1, **dadurch gekennzeichnet, daß** die Digitalisierseinrichtung vier Analog/Digital-Wandler (32-38) umfaßt, die jeweils eines der analogen Signale $X^+$, $X^-$, $Y^+$, und $Y^-$ verarbeiten.

3. Vorrichtung nach Anspruch 1, **dadurch gekennzeichnet, daß** die Digitalisiereinrichtung einen Multiplex-Analog/Digital-Wandler (44) zum sequentiellen Bearbeiten der analogen Signale $X^+$, $X^-$, $Y^+$, und $Y^-$ umfaßt.

4. Vorrichtung nach einem der Ansprüche 1 bis 3, **dadurch gekennzeichnet, daß** die digitale Recheneinrichtung (42) die digitalen Koordinatensignale $X_N$, $Y_N$ gemäß den Gleichungen

$$X_N = \frac{X_N^+ - X_N^-}{X_N^+ + X_N^-} \quad und \quad Y_N = \frac{Y_N^+ - Y_N^-}{Y_N^+ + Y_N^-}$$

erzeugt.

5. Vorrichtung nach einem der Ansprüche 1 bis 3, **dadurch gekennzeichnet, daß** die digitale Recheneinrichtung (42) die digitalen Koordinatensignale $X_N$, $Y_N$ gemäß den Gleichungen

$$X_N = \frac{X_N^+ - X_N^-}{X_N^+ + X_N^- + Y_N^+ + Y_N^-} \quad \text{und} \quad Y_N = \frac{Y_N^+ - Y_N^-}{X_N^+ + X_N^- + Y_N^+ + Y_N^-}$$

erzeugt.

6. Vorrichtung nach einem der Ansprüche 1 bis 3, **dadurch gekennzeichnet, daß** der Positionskodierer (18) außerdem eine Bewertungseinrichtung (30) zum Abgeben eines analogen Energie-Signals Z, welches gleich der Summe der von den Meßwertumsetzern (28) gelieferten elektrischen Signale ist, aufweist, wobei das Signal Z durch die Digitalisiereinrichtung des Positionskodierers in ein digitales Signal $Z_N$ umgewandelt wird.

7. Vorrichtung nach Anspruch 6, **dadurch gekennzeichnet, daß** die digitale Recheneinrichtung (42) digitale Koordinatensignale $X_N$, $Y_N$ gemäß den Gleichungen

$$X_N = \frac{X_N^+ - X_N^-}{Z_N^-} \quad \text{und} \quad Y_N = \frac{Y_N^+ - Y_N^-}{Z_N^-}$$

erzeugt.

8. Vorrichtung zur Erzeugung eines Strahlungsbildes mit einer Kernstrahlungs-Lokalisiereinrichtung (46), die digitale Koordinatensignale $X_N$, $Y_N$ abgibt, die die Position der empfangenen Kernstrahlung angeben, und mit einer Bilderzeugungseinrichtung (48), die einen Speicher, eine Anzeigeeinheit und eine Verarbeitungseinheit aufweist, die die digitalen Koordinatensignale $X_N$, $Y_N$ empfängt, die Anzahl der in jedem Punkt des Koordinatenbildes $X_N$, $Y_N$ empfangenen Kernstrahlungen in dem Speicher speichert und die Anzeigeeinheit steuert, so daß sie ein Strahlungsbild erzeugt, auf dem die Intensität jedes Bildpunktes von der Anzahl der empfangenen Kernstrahlungen abhängt, **dadurch gekennzeichnet, daß** die Kernstrahlungs-Lokalisierungseinrichtung gemäß einem der Ansprüche 1 bis 7 ausgebildet ist.

## Claims

1. Device for locating a nuclear radiation incorporating a detection head (16) for detecting a nuclear radiation (24) and a position coder (18) for locating said nuclear radiation, said detection head comprising a collimator (20), a planar layer (26) of a material sensitive to said nuclear radiation for receiving on a first or front face a nuclear radiation traversing said collimator and for supplying on a second or rear face a light radiation which is a function of the nuclear radiation received, a plurality of transducers (28) arranged on the rear face of said layer, each supplying an electric signal which is a function of the light radiation received, each nuclear radiation producing a light radiation seen by a plurality of transistors, wherein the position coder (18) comprises weighting means (30) for supplying analog electric signals $X^+$, $X^-$, $Y^+$, $Y^-$, each representing weighted sums of the electric signal supplied by the transducers, the signals $X^+$, $X^-$ being a function of the position and the energy of the nuclear radiation received in accordance with an axis X and the signals $Y^+$, $Y^-$ being a function of the position of the nuclear radiation received in accordance with an axis Y, characterized in that it also comprises a digitizing means (32-38, 44) for receiving the analog signals $X^+$, $X^-$, $Y^+$, $Y^-$ and for supplying corresponding digital signals $X_N^+$, $X_N^-$, $Y_N^+$ and $Y_N^-$ and a calculating means (42) or computer for determining the signals of digital coordinates $X_N$, $Y_N$ of the nuclear radiation received as a function of the digital signals $X_N^+$, $X_N^-$, $Y_N^+$ and $Y_N^-$.

2. Device according to claim 1, characterized in that the digitizing means comprises 4 analog-digital converters (32-38), each of which processes one of the analog signals $X^+$, $X^-$, $Y^+$, $Y^-$.

3. Device according to claim 1, characterized in that the digitizing means comprises a multiplexed analog-digital converter (44) for sequentially processing the analog signals $X^+$, $X^-$, $Y^+$, $Y^-$.

4. Device according to any one of the claims 1 to 3, characterized in that the digital computer (42) produces digital signals of coordinates $X_N$, $Y_N$ according to the relations :

$$X_N = \frac{X_N^+ - X_N^-}{X_N^+ + X_N^-} \quad \text{and} \quad Y_N = \frac{Y_N^+ - Y_N^-}{Y_N^+ + Y_N^-}$$

5. Device according to any one of the claims 1 to 3, characterized in that the digital computer (42) produces digital signals of coordinates $X_N$, $Y_N$ according to the relations :

$$X_N = \frac{X_N^+ - X_N^-}{X_N^+ + X_N^- + Y_N^+ + Y_N^-} \quad \text{and} \quad Y_N = \frac{Y_N^+ - Y_N^-}{X_N^+ + X_N^- + Y_N^+ + Y_N^-}$$

6. Device according to any one of the claims 1 to 3, characterized in that the position coder (8) also comprises a weighting means (30) for supplying an analog signal of energy Z equal to the sum of the electric signals supplied by the transducers (28), said signal Z being converted into a digital signal $Z_N$ by the digitizing means of the position coder.

7. Device according to claim 6, characterized in that the digital computer (42) produces digital signals of the coordinates $X_N$, $Y_N$ in accordance with relations :

$$X_N = \frac{X_N^+ + X_N^-}{Z_N} \quad \text{and} \quad Y_N = \frac{Y_N^+ - Y_N^-}{Z_N}$$

8. Radiation image formation device incorporating a nuclear radiation locating device (46) supplying digital signals of coordinates $X_N$, $Y_N$ indicating the position of a nuclear radiation received, and an image formation means (48) comprising a memory, a readout and a processing emans receiving said digital signals of coordinates $X_N$, $Y_N$, storing in said memory the number of nuclear radiations received at each image point of coordinates $X_N$, $Y_N$ and controlling the readout to produce a radiation image in which the intensity of each image point is a function of said number of received nuclear radiations, characterized in that said nuclear radiation locating device is in accordance with any one of the claims 1 to 7.

FIG. 1

FIG. 2

FIG. 3

FIG. 4

A

X⁺

X⁻

O

x

FIG. 5

N

B

140 keV

E

FIG. 6

O

X

A

00  01  10  11  100  101  ....

FIG. 7

FIG. 8

FIG 9